# EUROPEAN PATENT APPLICATION

(11) **EP 4 309 622 A1**
(43) Date of publication of application: **24.01.2024**
(21) Application number: 22771819.4
(22) Date of filing: 18.03.2022
(51) Int. Cl.: A61C 19/05, A61C 9/00, G16H 50/50, G06T 19/20, G16H 30/00

(54) **DATA PROCESSING METHOD**

(30) Priority: 19.03.2021 KR 20210035960
(71) Applicant: Medit Corp., Seoul 07207 (KR)
(72) Inventor: LEE, Dong Hoon, Seoul 02842 (KR); NAM, Ha Jong, Seoul 02842 (KR)
(74) Representative: Maiwald GmbH
(86) International application number: PCT/KR2022/003837
(87) International publication number: WO 2022/197153

(57) **Abstract**

A data processing method according to the present invention comprises: an occlusion data acquisition step of acquiring a plurality of occlusion data, which includes at least a portion of the shape of the upper jaw and the shape of the lower jaw of an object; an occlusion model generation step of aligning each of the plurality of occlusion data with upper jaw data expressing the upper jaw and lower jaw data expressing the lower jaw, so as to generate a plurality of occlusion models; and an occlusion model display step of continuously displaying at least some of the plurality of occlusion models.

## Description

### TECHNICAL FIELD

The present disclosure relates to a data processing method and, more particularly, to a data processing method of acquiring a plurality of occlusion models and easily identifying differences between the respective occlusion models.

### BACKGROUND

3D scanning technology is being used in various industries such as measurement, inspection, reverse engineering, content creation, CAD/CAM for dental treatment, medical devices, and the like, and the improvement of scanning performance due to the development of computing technology is further expanding the practicality thereof. In particular, since the 3D scanning technology is performed for patient treatment in the field of dental treatment, a 3D model acquired through the 3D scanning is required to have high precision.

In the process of generating a 3D model through a 3D scanner, the 3D scanner converts (2D or 3D) image data acquired by photographing an object to be measured into a 3D model, thereby acquiring the entire 3D model. In the field of dental treatment, it is possible to acquire a 3D model expressing the inside of the patient's mouth by scanning the inside of the patient's mouth and to provide optimal treatment to the patient by analyzing the 3D model. Meanwhile, a 3D model (hereinafter, which may be referred to as an occlusion model in the present disclosure) representing the inside of the patient's mouth may be acquired by acquiring upper jaw data representing the patient's upper jaw, lower jaw data representing the patient's lower jaw, and occlusion data representing one buccal side of the upper jaw and lower jaw and aligning them with each other.

Meanwhile, in order to provide optimal treatment to the patient, occlusion data that represents various occlusion aspects such that the patient's upper jaw and lower jaw occlude with each other is required. For example, a user of a 3D scanner may acquire first occlusion data representing aperture occlusion, second occlusion data representing lateral occlusion, and third occlusion data representing cutting occlusion. By acquiring a plurality of occlusion data, a plurality of 3D occlusion models may be generated on the basis of the plurality of occlusion data. Accordingly, a method for analyzing the movement trajectory of a object using the plurality of generated occlusion models and providing proper treatment (e.g., providing a prosthetic product, etc.) to a patient is being studied.

### SUMMARY

The present disclosure provides a data processing method of generating a plurality of occlusion models by aligning a plurality of occlusion data with upper jaw data and lower jaw data and continuously displaying at least some of the plurality of occlusion models, thereby improving the user's visual convenience in the process of analyzing the plurality of occlusion models through an animation effect according to switching between the plurality of occlusion models.

The technical problems to be solved by the present disclosure are not limited to the technical problems mentioned above, and other technical problems not mentioned will be clearly understood by those skilled in the art from the description below.

In order to attain the objective described above, a data processing method according to the present disclosure may include an occlusion data acquisition step of acquiring a plurality of occlusion data including at least a portion of a shape of an upper jaw and a shape of a lower jaw of a object, an occlusion model generation step of generating a plurality of occlusion models by aligning each of the plurality of occlusion data with upper jaw data representing the upper jaw and lower jaw data representing the lower jaw, and an occlusion model display step of continuously displaying at least some of the plurality of occlusion models.

In addition, a data processing method according to the present disclosure may further include other additional steps in addition to the above-described steps, enabling a user to easily acquire and compare a plurality of occlusion models.

By using the data processing method according to the present disclosure, there is an advantage that makes it possible for the user to acquire a plurality of occlusion models aligned by a plurality of occlusion data and easily compare the plurality of occlusion models.

In addition, by using the data processing method according to the present disclosure, there is an advantage that makes it possible for the user to visually and conveniently compare and analyze a plurality of occlusion models through an animation step in which lower jaw data moves while upper jaw data of the plurality of occlusion models is fixed.

In addition, in the data processing method according to the present disclosure, since the occlusion data is aligned with the upper jaw data and since the lower jaw data is aligned with the occlusion data, it is possible to acquire accurate occlusion models according to the stable alignment, and since the movement of the upper jaw data and/or the lower jaw data is effectively displayed to the user through an animation step, there is an advantage of improving the user's visual convenience. In particular, in the case of acquiring the occlusion models by aligning the upper jaw data, the occlusion data, and the lower jaw data while the upper jaw data is fixed, it is possible to acquire accurate occlusion models according to stable alignment, and since the lower jaw data is quickly aligned through the occlusion data, the speed of acquiring the plurality of occlusion models is increased, and since the movement of the upper jaw data and/or lower jaw data is effectively displayed to the user through an animation step of continuously displaying the plurality of occlusion models, there is an advantage of improving the user's visual convenience.

In addition, the positions of the upper jaw data and the lower jaw data are returned to the positions before the alignment of the occlusion data or are spaced by a predetermined distance from each other in an alignment initialization step of the data processing method according to the present disclosure, so that the upper jaw data and the lower jaw data can be aligned by new occlusion data to create a new occlusion model, and there is an advantage that makes it possible for the user to visually and easily recognize that the occlusion model aligned before the alignment initialization step has been safely stored by the alignment initialization step.

In addition, since a plurality of occlusion models is generated in a single stage in the data processing method according to the present disclosure, there is an advantage of omitting a stage switching process for the user to acquire a plurality of occlusion data and acquire respective occlusion models, and improving the user convenience.

In addition, in the data processing method according to the present disclosure, since a movement path is acquired to switch between a plurality of occlusion models and since the lower jaw data moves along the movement path, there is an advantage of visually and easily identifying the movement of a object through an animation effect.

In addition, since at least one interpolation model is generated according to the movement path for switching between a plurality of occlusion models and displayed sequentially in a predetermined order, differences between the plurality of occlusion models including the movement of the upper jaw data and the lower jaw data through an animation effect are displayed with maximized visuality, which has an advantage of improving the user's visual convenience.

In addition, since a reference occlusion model determined in a reference occlusion model determination step can be used as a reference for aligning other occlusion models, and since a plurality of occlusion models is aligned on the basis of the upper jaw data of the reference occlusion model, the user can visually and easily identify the movement of the plurality of occlusion models aligned on the basis of the reference occlusion model through an animation effect. In addition, there is an advantage that makes it possible to design accurate prosthetic products through a reference occlusion model expressing a normal bite.

In addition, when the upper jaw data of a plurality of occlusion models is aligned through upper jaw data of a reference occlusion model, since the movement path of the lower jaw data is acquired so that the movement of the lower jaw data is continuously displayed in the animation step, there is an advantage of quickly realizing the movement of data.

In addition, since a plurality of occlusion models can be generated by a combination of first upper jaw data and first lower jaw data representing the shapes of the upper jaw and lower jaw before processing the same, and second upper jaw data and second lower jaw data representing the shape of the upper jaw and lower jaw after processing the same, there is an advantage that makes it possible for the user to design accurate prosthetic products for the patient with reference to occlusion models representing the upper jaw data and lower jaw data in various states, thereby providing optimal treatment to the patient.

### BRIEF DESCRIPTION OF DRAWINGS

FIG. 1 is a block diagram of a data processing apparatus performing a data processing method according to an embodiment of the present disclosure.
FIG. 2 is a flowchart of a data processing method according to an embodiment of the present disclosure.
FIGS. 3A and 3B are diagrams illustrating various occlusion types acquired in a data processing method according to an embodiment of the present disclosure.
FIG. 4 is a detailed flowchart of an occlusion model generation step S 120 of a data processing method according to an embodiment of the present disclosure.
FIGS. 5 to 7 are diagrams illustrating a process of generating an occlusion model by aligning upper jaw data and lower jaw data using occlusion data in a data processing method according to an embodiment of the present disclosure.
FIG. 8 is a diagram illustrating an alignment initialization step S 123 of a data processing method according to an embodiment of the present disclosure.
FIG. 9 is a detailed flowchart of an occlusion model display step S 140 of a data processing method according to an embodiment of the present disclosure.
FIG. 10 is a diagram illustrating a plurality of occlusion models acquired by a data processing method according to an embodiment of the present disclosure.
FIG. 11 is a diagram illustrating a process of moving upper jaw data and/or lower jaw data according to switching between a plurality of occlusion models in a data processing method according to an embodiment of the present disclosure.
FIGS. 12 to 14 are diagrams illustrating a process in which an occlusion model moves by selecting different occlusion models in a data processing method according to an embodiment of the present disclosure.
FIG. 15 is a diagram illustrating an interpolation model generated between occlusion models and a process in which an animation step is performed according to the interpolation model in a data processing method according to an embodiment of the present disclosure.
FIG. 16 is a diagram illustrating a process in which occlusion analysis of a first occlusion model is performed to determine a reference occlusion model in a data processing method according to an embodiment of the present disclosure.
FIG. 17 is a diagram illustrating a process in which occlusion analysis of a second occlusion model is performed to determine a reference occlusion model in a data processing method according to an embodiment of the present disclosure.
FIG. 18 is a diagram illustrating a process in which occlusion analysis is performed for an arbitrary occlusion model in a data processing method according to an embodiment of the present disclosure.

### [Drawing Legend Insertion]

1: Data processing unit
10: Scan unit 20: Control unit
30: Display unit
S 110: Occlusion data acquisition step
S 120: Occlusion model generation step
S130: Reference occlusion model determination step
S 140: Occlusion model display step

### DETAILED DESCRIPTION

Hereinafter, some embodiments of the present disclosure will be described in detail with reference to the accompanying drawings. In connection with reference numerals of components in the drawings, like components are indicated by like reference numerals through the drawings. In addition, in describing an embodiment of the present disclosure, if it is determined that a detailed description of a related known configuration or function hinders understanding of the embodiment of the present disclosure, the detailed description will be omitted.

In describing the components of the embodiment of the present disclosure, the terms such as first, second, A, B, (a), and (b) may be used. These terms are used only to distinguish the component from other components, and the nature, order, or sequence of the corresponding component is not limited by the term. In addition, all terms including technical or scientific terms used herein have meanings that are generally understood by a person having ordinary knowledge in the art to which the present disclosure pertains, unless otherwise specified. Terms such as those defined in commonly used dictionaries should be interpreted as having a meaning consistent with the meaning in the context of the related art and, unless explicitly defined herein, should not be interpreted in an ideal or excessively formal meaning.

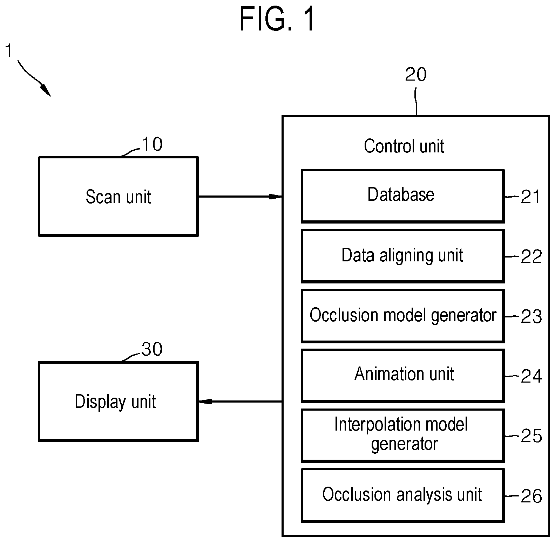

FIG. 1 is a block diagram of a data processing apparatus 1 that performs a data processing method according to an embodiment of the present disclosure.

Referring to FIG. 1, a data processing apparatus 1 performing a data processing method according to an embodiment of the present disclosure may include a scan unit 10, a control unit 20, and a display unit 30.

The scan unit 10 may scan an object and acquire data representing the object. In this case, the object may be the inside of the patient's actual mouth representing the patient's oral shape and oral condition. However, the object does not necessarily have to be the inside of the patient's actual mouth, and the object may be at least one of an impression model (intaglio model) made by imitating the patient's actual mouth with an impression material and a plaster model (embossed model) acquired by pouring plaster on the impression model, in addition to the inside of the patient's actual mouth described above. That is, any object representing the patient's oral shape and oral condition may function as an object.

The scan unit 10 may scan the object to acquire 2D image data that two-dimensionally expresses the shape and color of the object and a 3D model that three-dimensionally expresses the shape and color of the object (which may be referred to as an occlusion model in the present disclosure). In this case, the scan unit 10 may emit structured light toward the object in order to acquire depth information to generate a 3D model of the object. For example, the scan unit 10 may have a built-in light projector capable of emitting structured light. In addition, the structured light emitted from the light projector may be reflected on the surface of the object, and the reflected light may be received by the scan unit 10. For example, the reflected light reflected from the surface of the object may be received by a camera of the scan unit 10, thereby generating 2D image data and a 3D model based on the reflected light received by the camera.

The scan unit 10 may acquire a plurality of occlusion data including at least a part of the shape of an upper jaw and the shape of a lower jaw of the object by scanning the object. For example, the scan unit 10 may be a handheld 3D scan unit capable of carrying out a scanning process so as to have a free scan distance and free scan angle with respect to the object while the user holds the same. As another example, the scan unit 10 may be a table-type 3D scan unit capable of carrying out a scanning process by placing an object thereon and rotating and/or tilting the object.

The control unit 20 may be connected to and communicate with the scan unit 10 through wire or wireless communication. The control unit 20 and the scan unit 10 may perform data communication using a known communication method. The control unit 20 may process data acquired by the scan unit 10 scanning the object. For example, the control unit 20 may align occlusion data acquired by scanning an object by the scan unit 10 with upper jaw data and lower jaw data, thereby generating occlusion models, and implement an animation effect from one occlusion model to another occlusion model in the process of switching between a plurality of occlusion models. In addition, the control unit 20 may further generate an interpolation model for implementing the animation effect or determine a reference occlusion model by analyzing a plurality of occlusion models.

The control unit 20 may include a database 21. The database 21 may store data acquired from the scan unit 10. In addition, the database 21 may store generated occlusion models. In addition, the database 21 may store various logics for the operation of the control unit 20. For example, the database 21 may include at least one of a logic for aligning occlusion data, upper jaw data, and lower jaw data, a logic for aligning a plurality of occlusion models, a logic for acquiring a moving path for performing an animation step, a logic for generating an interpolation model corresponding to the movement path, and a logic for determining a reference occlusion model from among a plurality of occlusion models. The database 21 may be a known storage device. For example, the database 21 may be a storage device such as a hard disk drive, a solid state drive, or a USB flash drive. In addition, the database 21 may be a storage system in the form of a cloud.

The control unit 20 may include a data aligning unit 22. The data aligning unit 22 may align upper jaw data and lower jaw data through occlusion data. For example, if the scan unit 10 acquires occlusion data by scanning the buccal sides of the upper jaw and lower jaw of a object, the occlusion data may be aligned with the upper jaw data and lower jaw data, and the upper jaw data and lower jaw data may have an aligned shape. Meanwhile, if an occlusion model is generated by an occlusion model generator 23 to be described later, the data aligning unit 22 may release the application of the occlusion data to acquire a new occlusion model and return the positions of the upper jaw data and lower jaw data to the previous positions before aligning the same or space the upper jaw data and the lower jaw data from each other by a predetermined distance.

The control unit 20 may include an occlusion model generator 23. The occlusion model generator 23 may generate a 3D occlusion model on the basis of the upper jaw data, the lower jaw data, and the occlusion data aligned by the data aligning unit 22. Meanwhile, if a plurality of occlusion data are acquired from the scan unit 10, the occlusion model generator 23 may generate a plurality of occlusion models by aligning each of the plurality of occlusion data, the upper jaw data, and the lower jaw data. The occlusion model generator 23 may acquire a plurality of occlusion models aligned by a plurality of occlusion data in a single stage.

The control unit 20 may include an animation unit 24. When one occlusion model switches to another occlusion model, the animation unit 24 may acquire a movement path of data and move the data along the movement path. For example, the animation unit 24 may continuously display the movement of the lower jaw data from the first occlusion model to the second occlusion model along the movement path.

In addition, the control unit 20 may include an interpolation model generator 25. The interpolation model generator 25 may be generated based on a movement path between a plurality of occlusion models. For example, the interpolation model generator 25 may generate at least one interpolation model between the first occlusion model and the second occlusion model. The animation unit 24 may display the interpolation model generated by the interpolation model generator 25 between the first occlusion model and the second occlusion model, thereby continuously displaying the movement of data.

In addition, the control unit 20 may include an occlusion analysis unit 26. The occlusion analysis unit 26 may analyze a plurality of occlusion models generated by the occlusion model generator 23 to determine a reference occlusion model. For example, the occlusion analysis unit 26 may determine a reference occlusion model on the basis of the size of an occlusion area where the upper jaw data and the lower jaw data come into contact with each other in a plurality of occlusion models.

The display unit 30 may visually display at least some of the operations of the control unit 20 described above. The display unit 30 may display 2D image data and a 3D model acquired in the scanning process of the scan unit 10. In addition, the display unit 30 may display a process in which the occlusion data, the upper jaw data, and the lower jaw data are aligned. In addition, the display unit 30 may display an animation process in which data moves or the interpolation model is sequentially displayed when switching from one occlusion model to another occlusion model. In addition, the display unit 30 may display a process of determining a reference occlusion model according to the occlusion analysis. The display unit 30 may be a known visual display device, and at least one of visual display devices such as a monitor, a touch screen, and a tablet device may be used as the display unit 30.

Hereinafter, a data processing method according to an embodiment of the present disclosure will be described in detail.

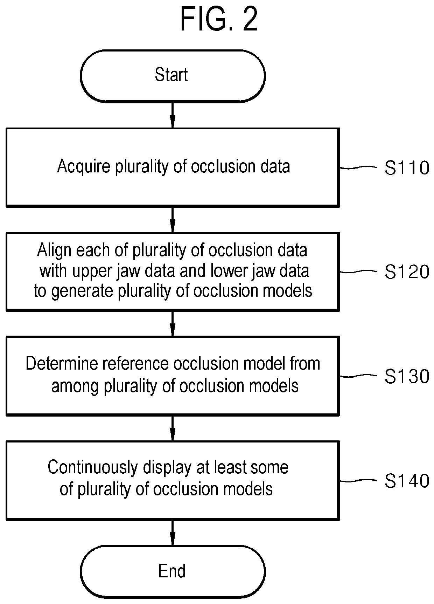

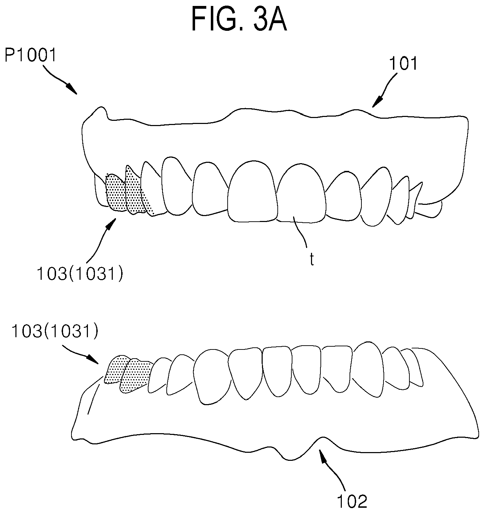

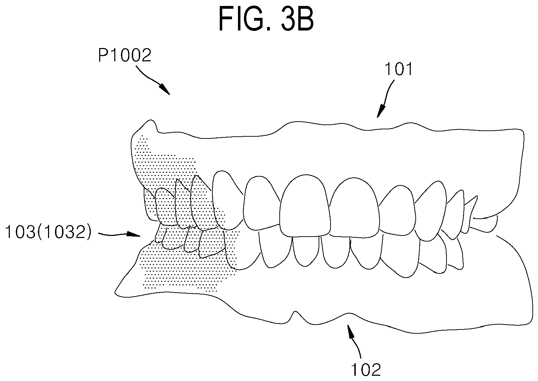

FIG. 2 is a flowchart of a data processing method according to an embodiment of the present disclosure, and FIGS. 3A and 3B are diagrams illustrating various occlusion types acquired in a data processing method according to an embodiment of the present disclosure.

Referring to FIG. 2, a data processing method according to an embodiment of the present disclosure may include an occlusion data acquisition step S110, an occlusion model generation step S120, and an occlusion model display step S140.

Hereinafter, the respective steps of the data processing method according to an embodiment of the present disclosure will be described in more detail.

Referring to FIGS. 2, 3A, and 3B, in an occlusion data acquisition step S 110, a scan unit may scan an object to acquire occlusion data. For example, in the occlusion data acquisition step S110, the scan unit may acquire a plurality of occlusion data including at least a portion of the shape of an upper jaw and the shape of a lower jaw of the object. The occlusion data may be acquired by scanning the buccal sides of the upper and lower jaws of the object. A plurality of occlusion data may be acquired to express different occlusion shapes when the upper jaw and lower jaw of the object occlude in different shapes.

For example, as shown in FIG. 3A, upper jaw data 101 including tooth data t and lower jaw data 102 including tooth data t may be opened, thereby acquiring an exemplary first occlusion model P1001 in which the upper jaw data 101 and the lower jaw data 102 are aligned by first occlusion data 103 and 1031 in an opening shape. In addition, as another example, as shown in FIG. 3B, upper jaw data 101 including tooth data t and lower jaw data 102 including tooth data t may occlude in the lateral direction, thereby acquiring an exemplary second occlusion model P1002 in which the upper jaw data 101 and the lower jaw data 102 are aligned by second occlusion data 103 and 1032 in a meshed shape. In this way, by acquiring a plurality of occlusion data 103 having various occlusion shapes, a plurality of occlusion models may be generated, and the user may analyze the patient's oral structure on the basis of the plurality of occlusion models to provide optimal treatment.

Meanwhile, in describing the present disclosure, the occlusion data 103 may include one side occlusion data acquired by scanning one side buccal sides of the upper jaw and lower jaw, and the other side occlusion data acquired by scanning the other side buccal sides of the upper jaw and lower jaw. However, in the present disclosure, it may be understood that the plurality of occlusion data 103 are acquired by scanning different occlusion shapes.

That is, a plurality of occlusion data 103 may be acquired by scanning a portion of a buccal side of an object having a plurality of different occlusion states. For example, the plurality of occlusion data 103 may include first occlusion data acquired by occluding the object in a first occlusion state and second occlusion data acquired by occluding the object in a second occlusion state, and a plurality of occlusion models may be acquired based on a plurality of occlusion data representing the respective occlusion shapes, so that the user may compare and analyze the plurality of occlusion models.

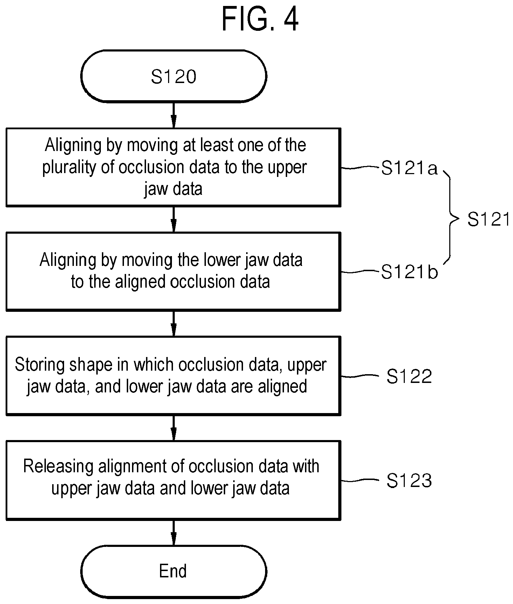

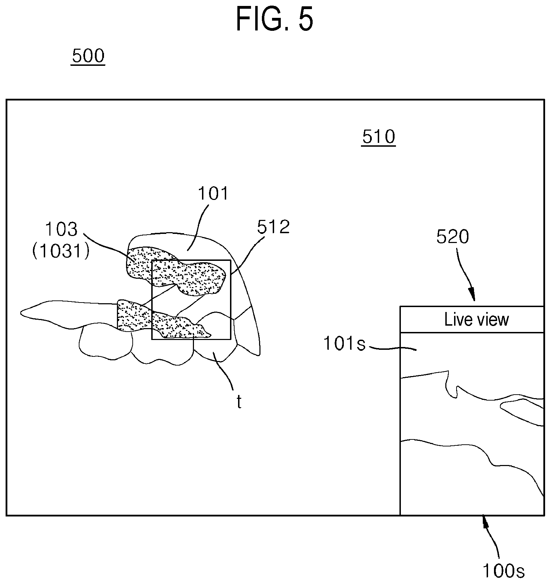

FIG. 4 is a detailed flowchart of an occlusion model generation step S 120 of a data processing method according to an embodiment of the present disclosure. In addition, FIGS. 5 to 7 are diagrams illustrating a process of generating an occlusion model 100 by aligning upper jaw data 101 and lower jaw data 102 using occlusion data 103 in a data processing method according to an embodiment of the present disclosure.

Referring to FIGS. 2 and 4, in the data processing method according to an embodiment of the present disclosure, an occlusion model generation step S120 may be performed after an occlusion data acquisition step S 110. For example, the control unit communicating with the scan unit may align occlusion data acquired from the scan unit with upper jaw data and lower jaw data. The upper jaw data representing the upper jaw of an object and the lower jaw data representing the lower jaw of the object may be acquired by the scan unit prior to the occlusion data acquisition step S1 10. In addition, the upper jaw data and the lower jaw data may be prestored in the database of the control unit and loaded to generate an occlusion model. Meanwhile, the upper jaw data and the lower jaw data do not necessarily have to be acquired before the occlusion data acquisition step S 110 and may be acquired between the occlusion data acquisition step S110 and the occlusion model generation step S120 as necessary. In this case, at least one piece of the upper jaw data and the lower jaw data may be acquired. However, it is assumed that one piece of upper jaw data and one piece of lower jaw data have been previously acquired, respectively, for convenience of description in describing the present disclosure.

For example, in the occlusion model generation step S 120, each of the plurality of occlusion data acquired in the occlusion data acquisition step S 110 may be aligned with the upper jaw data and the lower jaw data, thereby generating a plurality of occlusion models. The plurality of generated occlusion models may be continuously displayed in the occlusion model display step S 140 to be described later, and the user may easily identify the movement of the upper jaw data and/or lower jaw data according to switching between the occlusion models, thereby improving the user's visual convenience.

In the occlusion model generation step S 120, the upper jaw data, the lower jaw data, and the occlusion data may be aligned according to a predetermined rule. For example, in the occlusion model generation step S120, the upper jaw data and the lower jaw data may be aligned through the occlusion data while the upper jaw data and the lower jaw data are not fixed. As another example, in the occlusion model generation step S120, in the state in which one of the upper jaw data 101 and the lower jaw data 102 is fixed, the reaming one may be aligned toward the fixed data side. For example, in the occlusion model generation step S120, in the state in which the upper jaw data is fixed, the lower jaw data may be aligned toward the fixed upper jaw data side. In this case, at least one piece of occlusion data among the plurality of occlusion data may move toward the fixed upper jaw data and then be aligned with the upper jaw data, and the lower jaw data may move toward the occlusion data aligned with the upper jaw data and then be aligned with the occlusion data. As another example, in the occlusion model generation step S120, in the state in which the lower jaw data is fixed, the upper jaw data may be aligned toward the fixed lower jaw data side. In this case, at least one piece of occlusion data among the plurality of occlusion data may move toward the fixed lower jaw data and the be aligned with the lower jaw data, and the upper jaw data may move toward the occlusion data aligned with the lower jaw data and then be aligned with the occlusion data.

When the upper jaw data and the lower jaw data are aligned by the occlusion data in the occlusion model generation step S120, the fixed data may be the upper jaw data. In general, the movement of the lower jaw is greater than that of the upper jaw in the patient's oral structure. Therefore, in the state in which the upper jaw data representing the upper jaw of the object is fixed, the lower jaw data is aligned with the fixed upper jaw data, so that the movement of the lower jaw data may be easily expressed in the occlusion model display step S140 to be described later.

The occlusion model generation step S120 will be described in more detail. The occlusion model generation step S120 may include an oral data alignment step S121. In the oral data alignment step S121, the data aligning unit of the control unit may align at least one of the plurality of occlusion data, the upper jaw data, and the lower jaw data. Through the oral data alignment step S121, the upper jaw data and the lower jaw data may be aligned to occlude according to the shape of the occlusion data.

The oral data alignment step S121 may include a first alignment step S121a. For example, in the first alignment step S121a, at least one of the plurality of occlusion data acquired in the occlusion data acquisition step S110 may be moved toward the upper jaw data side and aligned therewith.

Referring to FIG. 5, a process of aligning arbitrary occlusion data 103, upper jaw data 101, and lower jaw data 102 will be described. As shown in FIG. 5, a screen of a user interface 500 displayed by the display unit is shown. The screen of the user interface 500 may include a model display portion 510 and a real-time display portion 520. The model display portion 510 may display at least one piece of occlusion data 103 acquired in the occlusion data acquisition step S110, upper jaw data 101, and lower jaw data 102. In addition, the real-time display portion 520 may display an image (e.g., 2D image data) acquired by the scan unit in real time. The real-time display portion 520 may display an image of a portion corresponding to a scan area 512 displayed on the model display portion 510 in real time. For example, the real-time display portion 520 may display an object image 100s including a 2D upper jaw image 101s in real time.

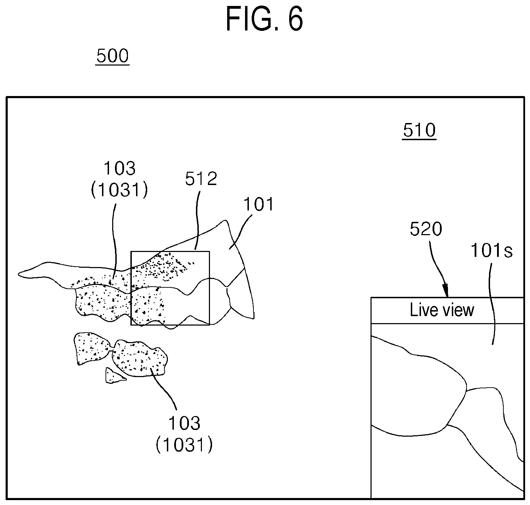

Referring to FIGS. 5 and 6, the scan unit may scan the buccal side of an object to acquire first occlusion data 1031, and the acquired first occlusion data 1031 may be displayed on the model display portion 510 in the first alignment step S121a. In this case, the first occlusion data 1031 may be moved toward the upper jaw data 101 side so that the first occlusion data 1031 and the upper jaw data 101 may be aligned. The first occlusion data 1031 may be displayed on the model display portion 510 to have a first pattern and/or a first color before being aligned with at least one piece of the upper jaw data 101 and the lower jaw data 102. For example, first occlusion data 1031 that is not aligned with any one piece of the upper jaw data 101 and the lower jaw data 102 may be displayed in gray. As shown in FIG. 6, the first occlusion data 1031 may be moved toward and aligned with the upper jaw data 101, and the first occlusion data 1031 aligned with the upper jaw data 101 may be displayed on the model display portion 510 to have a second pattern and/or a second color. For example, the first occlusion data 1031 aligned with the upper jaw data 101 may be displayed in light green.

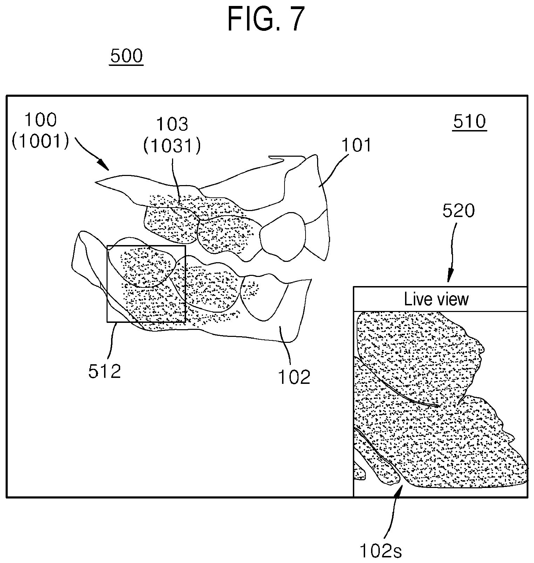

In addition, the oral data alignment step S121 may include a second alignment step S121b. For example, in the second alignment step S121b, the lower jaw data 102 may be moved toward and aligned with the occlusion data 103 side that is aligned with the upper jaw data 101. Referring to FIG. 7, the lower jaw data 102 may move toward the first occlusion data 1031 side so as to be aligned with the first occlusion data 1031 that is aligned with the upper jaw data 101. When the lower jaw data 102 is aligned with the first occlusion data 1031, the first occlusion data 1031 aligned with both the upper jaw data 101 and the lower jaw data 102 may be displayed on the model display portion 510 to have a third pattern and/or a third color. For example, the first occlusion data 1031 aligned with the upper jaw data 101 and the lower jaw data 102 may be displayed in green.

In the aforementioned oral data alignment step S121, the occlusion data 103, the upper jaw data 101, and the lower jaw data 102 may be aligned using a known data alignment method. For example, in the oral data alignment step S121, the occlusion data 103, the upper jaw data 101, and the lower jaw data 102 may be aligned using an iterative closest point (ICP) algorithm, but not necessarily limited thereto, and any suitable alignment method may be used to align the data.

If the oral data alignment step S121 is performed as described above, an occlusion model storage step S122 may be performed. The occlusion model generator of the control unit may generate an occlusion model 100 through a shape in which the upper jaw data 101 and the lower jaw data 102 are aligned by any one occlusion data 103 of a plurality of occlusion data, and the generated occlusion model 100 may be stored in the database of control unit. In the occlusion model storage step S 122, the plurality of occlusion data acquired in the occlusion data acquisition step S 110 may be aligned with the upper jaw data 101 and the lower jaw data 102 to generate and store a plurality of occlusion models 100, and the plurality of occlusion models 100 may represent various occlusion states of the patient. The user may provide the optimal treatment to the patient by analyzing the movement trajectory and occlusion state according to switching between the plurality of occlusion models 100.

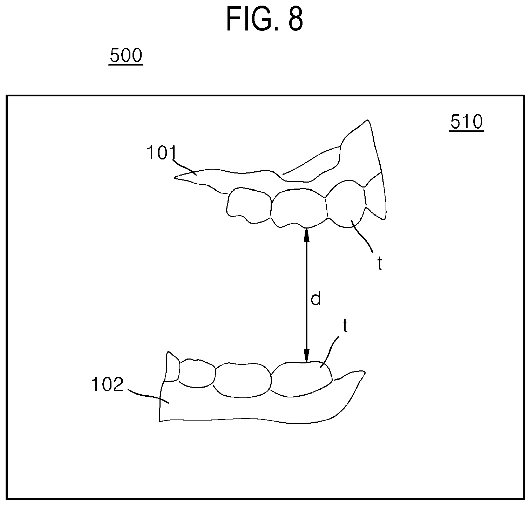

FIG. 8 is a diagram illustrating an alignment initialization step S123 of a data processing method according to an embodiment of the present disclosure.

Referring to FIGS. 4 and 8, an occlusion model generation step S120 of a data processing method according to an embodiment of the present disclosure may include an alignment initialization step S123. In the alignment initialization step S123, the alignment of the occlusion data 103 with the upper jaw data 101 and lower jaw data 102 may be released. For example, the alignment of the occlusion data 1031 with the upper jaw data 101 and lower jaw data 102 may be released after the upper jaw data 101 and the lower jaw data 102 are aligned by the first occlusion data 1031 and after the first occlusion model is generated and stored according thereto. If new occlusion data 103 representing an occlusion state different from the occlusion state represented by the first occlusion data 1031 is acquired by the releasing of the alignment of the occlusion data 1031 with the upper jaw data 101 and lower jaw data 102, the upper jaw data 101 and the lower jaw data 102 may be aligned by the new occlusion data 103, thereby generating a second occlusion model.

For example, in the alignment initialization step S123, the upper jaw data 101 and the lower jaw data 102 may return to the previous positions before being aligned by the occlusion data 103. In the alignment initialization step S123, the upper jaw data 101 and the lower jaw data 102 may be spaced apart from each other by a predetermined distance so as to have a predetermined distance d between the upper jaw data 101 and the lower jaw data 102. In this case, as the upper jaw data 101 and the lower jaw data 102 are spaced apart from each other by the predetermined distance d, the tooth data t included in the upper jaw data 101 and the tooth data t included in the lower jaw data 102 may also be spaced apart from each other. As the alignment initialization step S123 is performed, when the new occlusion data 103 is acquired, the upper jaw data 101 and the lower jaw data 102 may be aligned by the new occlusion data 103, and there is an advantage in which the user may acquire a plurality of occlusion models 100 by acquiring new occlusion models aligned by the new occlusion data 103.

In addition, in the alignment initialization step S123, the lower jaw data 102 may be spaced apart from the upper jaw data 101 by a predetermined distance d while the position of the upper jaw data 101 is fixed. If the lower jaw data 102 is spaced apart to have a predetermined distance d in the state in which the upper jaw data 101 is fixed, the upper jaw data 101 and the lower jaw data 102 may be aligned at a certain position by a plurality of occlusion data 103, so there is an advantage of reducing the computational load of a system for implementing the present disclosure (a data processing device according to an embodiment of the present disclosure) because a separate alignment process of the upper jaw data 101 may be omitted in an occlusion model display step S140 to be described later.

In addition, there is an advantage in which the user is able to easily identify the relative position of the lower jaw data 102 with respect to the fixed upper jaw data 101 when acquiring the correction model 100 because the position of the upper jaw data 101 is fixed in the alignment initialization step S123. In addition, there is an advantage in which the user is able to easily and visually recognize that the occlusion model storage step S122 has been performed as the alignment initialization step S123 is performed and in which the user is able to quickly acquire occlusion data representing a new occlusion state of the object.

Meanwhile, the above-described occlusion model generation step S120 may be repeatedly performed whenever the upper jaw data 101 and the lower jaw data 102 are aligned by applying each of the plurality of occlusion data 103. That is, first occlusion data may be applied to the upper jaw data 101 and the lower jaw data 102 so that the upper jaw data 101 and the lower jaw data 102 may be aligned to generate a first occlusion model. Thereafter, the alignment of the first occlusion data is released, and second occlusion data may be applied to the upper jaw data 101 and the lower jaw data 102 so that the upper jaw data 101 and the lower jaw data 102 may be aligned to generate a second occlusion model. As described above, since the occlusion model generation step is repeatedly performed whenever the upper jaw data 101 and the lower jaw data 102 are aligned, there is an advantage in which a plurality of occlusion models 100 can be acquired and in which the user is able to analyze a variety of data using the plurality of occlusion models 100.

In addition, the plurality of occlusion models 100 may be generated in a single stage on the user interface 500 as a plurality of occlusion data 103 are acquired. For example, the user may acquire the upper jaw data 101 in an upper jaw data acquisition stage and acquire the lower jaw data 102 in a lower jaw data acquisition stage. After that, the user may acquire a plurality of occlusion data 103 in an occlusion data acquisition stage, thereby acquiring a plurality of occlusion models 100 in which the upper jaw data 101 and the lower jaw data 102 are aligned with each piece of occlusion data 103. In this case, the occlusion data acquisition stage is not divided into a first occlusion data acquisition stage, a second occlusion data acquisition stage, and a third occlusion data acquisition stage, and the user may easily acquire a plurality of occlusion models 100 just by acquiring a plurality of different occlusion data 103 by scanning the buccal side of the object. That is, there is an advantage of improving the user's scanning concentration and improving user convenience due to reduction in the time and effort required to acquire a plurality of occlusion models 100 because a plurality of occlusion data 103 are able to be acquired in a single occlusion data acquisition stage without switching a plurality of occlusion model stages.

Hereinafter, an occlusion model display step S140 will be described in detail.

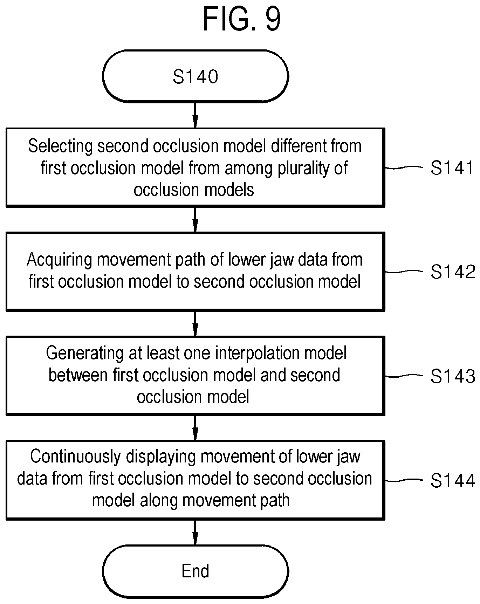

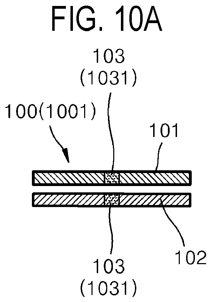

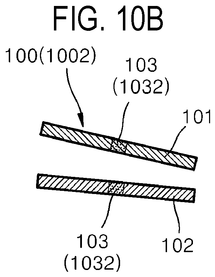

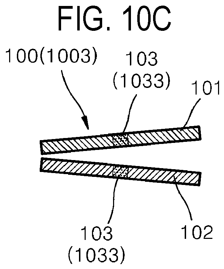

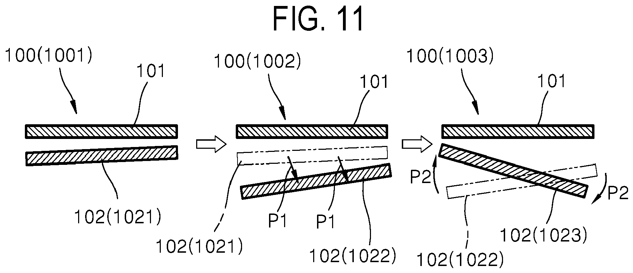

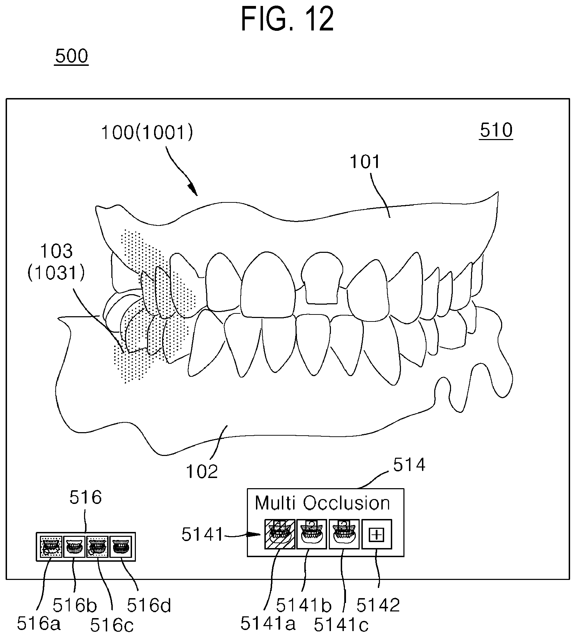

FIG. 9 is a detailed flowchart of an occlusion model display step S140 of a data processing method according to an embodiment of the present disclosure, FIG. 10 is a diagram illustrating a plurality of occlusion models 100 acquired by a data processing method according to an embodiment of the present disclosure, and FIG. 11 is a diagram illustrating a process of moving upper jaw data 101 and/or lower jaw data 102 according to switching between a plurality of occlusion models 100 in a data processing method according to an embodiment of the present disclosure. In addition, FIGS. 12 to 14 are diagrams illustrating a process in which an occlusion model 100 moves by selecting different occlusion models 100 in a data processing method according to an embodiment of the present disclosure.

Referring to FIGS. 2 and 9, a data processing method according to an embodiment of the present disclosure may include an occlusion model display step S140. In the occlusion model display step S140, the animation unit of the control unit may continuously display at least some of a plurality of occlusion models 100 acquired in the occlusion model generation step S120.

Referring to FIG. 10, exemplary occlusion models 100 are illustrated to explain the occlusion model display step S140. For example, in the occlusion model generation step S120, a first occlusion model 1001 aligned by first occlusion data 1031 may be shown in FIG. 10A, a second occlusion model 1002 aligned by second occlusion data 1032 may be shown in FIG. 10B, and a third occlusion model 1003 aligned by third occlusion data 1033 may be shown in FIG. 10C. As shown in FIG. 10, a plurality of occlusion models 100 may represent different occlusion states. There is an advantage in which the user is able to easily and visually identify the patient's oral structure through an animation process of implementing the movement of the upper jaw data 101 and/or the lower jaw data 102 according to switching between the plurality of occlusion models 100 depending on the cases, thereby improving the user's visual convenience. Accordingly, the user may provide optimal treatment to the patient by designing a prosthetic product suitable for the patient.

Referring to FIGS. 9 and 11, in order to effectively express the movement trajectories of the plurality of occlusion models 100, at least some of the plurality of occlusion models 100 may be aligned. That is, after the plurality of occlusion models 100 is generated, based on a predetermined portion of any one occlusion model 100 among the plurality of occlusion models 100, corresponding portions of the remaining occlusion models 100 may be aligned. For example, based on upper jaw data 101 of the first occlusion model 1001, upper jaw data 101 of the second occlusion model 1002 and upper jaw data 101 of the third occlusion model 1003 may be aligned. In this case, the first occlusion model 1001 may function as a reference occlusion model. In view of the anatomical structure of the mouth, in a plurality of occlusion models 100 having various occlusion shapes, a positional change of the upper jaw data 101 may be less than a positional change of the lower jaw data 102. Therefore, by aligning the upper jaw data 10 of the plurality of occlusion models 100, quick alignment between the plurality of occlusion models 100 is possible, and differences between the plurality of occlusion models including the movement of the upper jaw data and lower jaw data may be visually displayed to be maximized through an animation effect according to switching between the occlusion models 100, thereby improving the user's visual convenience and providing the user with optimal treatment.

The occlusion model display step S140 may include a switching model selection step S141 of selecting a second occlusion model 1002 different from the first occlusion model 1001 from among the plurality of occlusion models 100. For example, when the first occlusion model 1001 is displayed on the model display portion 510 of the screen of the user interface 500, the user may select an occlusion model 100 different from the first occlusion model 1001 such that the different occlusion models 100 is to be displayed on the model display portion 510. For example, when the first occlusion model 1001 is displayed on the model display portion 510, the user may select the second occlusion model 1002 to display the second occlusion model 1002 on the model display portion 510.

If the second occlusion model 1002 is selected in the switching model selection step S141, a movement path acquisition step S142 may be performed. In the movement path acquisition step S142, a movement path of data from the first occlusion model 1001 to the second occlusion model 1002 may be acquired. For example, in the movement path acquisition step 142, a movement path of the lower jaw data 102 from the first occlusion model 1001 to the second occlusion model 1002 may be acquired in the state in which the upper jaw data 101 of the plurality of occlusion models 100 is aligned and fixed. For example, the movement path may be a straight path along which the lower jaw data 101 of the first occlusion model 1001 moves to the position of the lower jaw data 102 of the second occlusion model 1002. As another example, the movement path may be acquired according to the anatomical joint movements of the upper jaw data 101 and the lower jaw data 102. In this case, at least one rotational center for rotating the lower jaw data 102 may be generated, and the movement path may be a curved path. In addition, the movement path may be a combination of a straight line and a curve.

Referring to FIGS. 9 and 11 to 13, if the movement path of the lower jaw data 102 is acquired according to the movement path acquisition step S142, an animation step S144 may be performed. In the animation step S144, the animation unit of the control unit may continuously display the movement of the lower jaw data 102 from the first occlusion model 1001 to the second occlusion model 1002 along the movement path. As shown in FIG. 11, when switching from the first occlusion model 1001 to the second occlusion model 1002, first lower jaw data 1021 of the first occlusion model 1001 may move toward the position of second lower jaw data 1022 of the second occlusion model 1002 along a first movement path P1.

For example, in the animation step S144, the movement speed of data may be constant. In this case, if the movement path when switching from the first occlusion model 1001 to the second occlusion model 1002 is long, the lower jaw data 102 may move for a relatively long time, and if the movement path is short, the lower jaw data 102 may move in a relatively short time. Accordingly, the user may compare the lengths of the movement paths between the occlusion models 100 through the movement time of the lower jaw data 102.

As another example, the animation step S144 may be performed for a constant period of time. That is, if the movement path is long, the lower jaw data 102 may move relatively quickly, and if the movement path is short, the lower jaw data 102 may move relatively slowly. Accordingly, the user may compare the lengths of the movement paths between the occlusion models 100 through the movement speed of the lower jaw data 102.

To the same effect, when switching from the second occlusion model 1002 to the third occlusion model 1003, the second lower jaw data 1022 of the second occlusion model 1002 may move toward the position of third lower jaw data 1023 of the third occlusion model 1003 along a second movement path P2. As described above, as the lower jaw data 102 moves along the movement path by switching between the respective occlusion models 100, the user may easily analyze the patient's oral structure and provide the patient with optimal treatment.

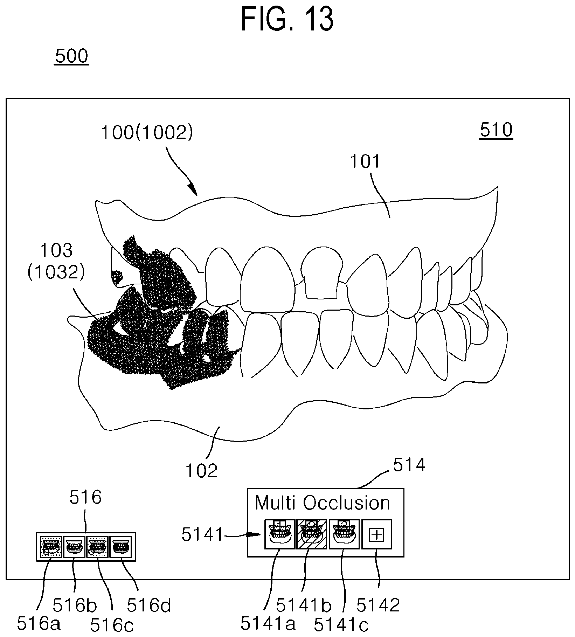

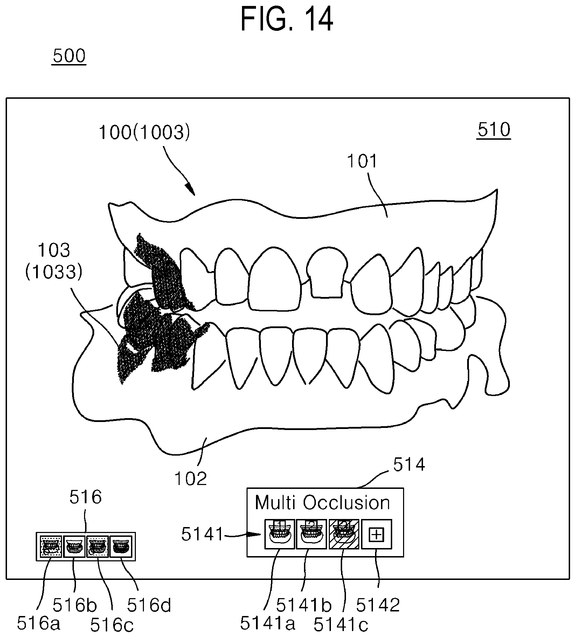

Hereinafter, the occlusion model display step S140 will be described with reference to the screen of the user interface 500. As shown in FIGS. 11 to 14, the model selection portion 510 on the screen of the user interface 500 may include a multi-occlusion menu 514. The multi-occlusion menu 514 may include multiple occlusion selection portions 5141, and the multiple occlusion selection portions 5141 may be formed as many as the number of occlusion models 100 generated and stored by performing the occlusion model generation step S120. For example, the multiple occlusion selection portions 5141 may include a first occlusion model selection portion 5141a for selecting the first occlusion model 1001, a second occlusion model selection portion 5141b for selecting the second occlusion model 1002, and a third occlusion model selection portion 5141c for selecting the third occlusion model 1003. The user may select any one of the first occlusion model selection portion 5141a, the second occlusion model selection portion 5141b, and the third occlusion model selection portion 5141c to switch the occlusion model 100 displayed on the model display portion 510. In the state where the first occlusion model 1001 is displayed on the model display portion 510 as shown in FIG. 12, the user may select the second occlusion model selection portion 5141b such that the second occlusion model 1002 is displayed on the model display portion 510 as shown in FIG. 13. Meanwhile, when the second occlusion model 1002 is displayed on the model display portion 510, the movement of the lower jaw data 102 may be continuously displayed. In addition, in the state where the second occlusion model 1002 is displayed on the model display portion 510 as shown in FIG. 13, the user may select the third occlusion model selection portion 5141c such that the third occlusion model 1003 is displayed on the model display portion 510 as shown in FIG. 14. When the third occlusion model 1003 is displayed on the model display portion 510, the movement of the lower jaw data 102 may be continuously expressed.

Although it has been described that the first occlusion model 1001 switches to the second occlusion model 1002 and that the second occlusion model 1002 switches to the third occlusion model 1003, the disclosure is not necessarily limited thereto. That is, the movement of data from the first occlusion model 1001 to the third occlusion model 1003 may be identified through an animation effect by selecting the third occlusion model 1003 while the first occlusion model 1001 is displayed, and the movement of data from the third occlusion model 1003 to the second occlusion model 1002 may be identified through an animation effect by selecting the second occlusion model 1002 while the third occlusion model 1003 is displayed.

In addition, the user may select an occlusion addition selection portion 5142 to acquire an additional occlusion model 100 from the multi-occlusion menu 514, thereby further acquiring a fourth occlusion model (not shown), and the user may easily and visually identify the movement of data according to switching among the first occlusion model to the fourth occlusion model through an animation effect, thereby improving the user's visual convenience.

Meanwhile, since the occlusion model display step (more specifically, the animation step) for implementing the movement of data according to the switching between the occlusion models is performed through the multi-occlusion menu 514 in a single stage, the user may easily and visually compare and analyze the plurality of occlusion models 100, thereby improving the user convenience.

Hereinafter, an interpolation model generation step S143 of the occlusion model display step S140 will be described.

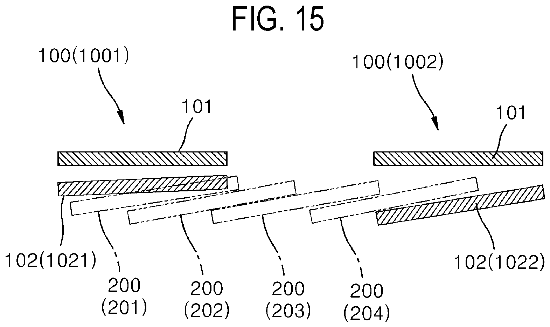

FIG. 15 is a diagram illustrating an interpolation model 200 generated between occlusion models 100 and a process in which an animation step S144 is performed according to the interpolation model 200 in a data processing method according to an embodiment of the present disclosure.

Referring to FIGS. 9 and 15, the occlusion model display step S140 may further include an interpolation model generation step S143. The interpolation model generator of the control unit may generate at least one interpolation model 200 between the second occlusion model 1002 and the first occlusion model 1001 selected in the switching model selection step S141. For example, in the interpolation model generation step S143, a first interpolation model 201, a second interpolation model 202, a third interpolation model 203, and a fourth interpolation model 204 may be generated based on the movement path from the first occlusion model 1001 to the second occlusion model 1002. In this case, the upper jaw data 101 of the first occlusion model 1001 and the second occlusion model 1002 may be fixed, and the interpolation model 200 may be generated for the lower jaw data 102.

In the animation step S144, the first occlusion model 1001, at least one interpolation model 200, and the second occlusion model 1002 may be sequentially displayed in a predetermined order according to the generated interpolation models 200 so that the movement of data (e.g., lower jaw data) from the first occlusion model 1001 to the second occlusion model 1002 may be continuously displayed. The user may easily and visually identify the movement of data according to the switching between the occlusion models 100 through an animation effect by generating at least one interpolation model 200 and displaying the movement of data through the animation effect. As the number of interpolation models 200 increases, the movement of the lower jaw data may be expressed smoothly in the animation step S144. Meanwhile, as the interpolation model 200 is generated, the user may further identify the oral structure at a specific time between the first occlusion model 1001 and the second occlusion model 1002, and the user may analyze the patient's oral structure in more detail by the interpolation model 200.

Hereinafter, a reference occlusion model determination step S130, which is an additional step of a data processing method according to an embodiment of the present disclosure, will be described.

Referring to FIGS. 2 and 11, a data processing method according to an embodiment of the present disclosure may further include a reference occlusion model determination step S130 after the occlusion model generation step S120. The reference occlusion model determination step S130 may indicate determining a reference occlusion model from among a plurality of occlusion models 100 generated in the occlusion model generation step S120. The reference occlusion model may refer to an occlusion model 100 to be preferentially considered in order to analyze the patient's oral structure. For example, the reference occlusion model may represent a normal bite.

If the reference occlusion model is determined, other occlusion models 100 may be aligned with respect to the reference occlusion model. For example, if the first occlusion model 1001 is determined from among three occlusion models 100 as a reference occlusion model, the second occlusion model 1002 and the third occlusion model 1003 may be aligned with respect to the first occlusion model 1001 that is the reference occlusion model. For example, in the plurality of occlusion models 100, based on a predetermined portion of the reference occlusion model, corresponding portions of the remaining occlusion models 100 may be aligned. More specifically, the upper jaw data 101 of the second occlusion model 1002 and the upper jaw data 101 of the third occlusion model 1003 may be aligned based on the upper jaw data 101 of the first occlusion model 1001, which is a reference occlusion model.

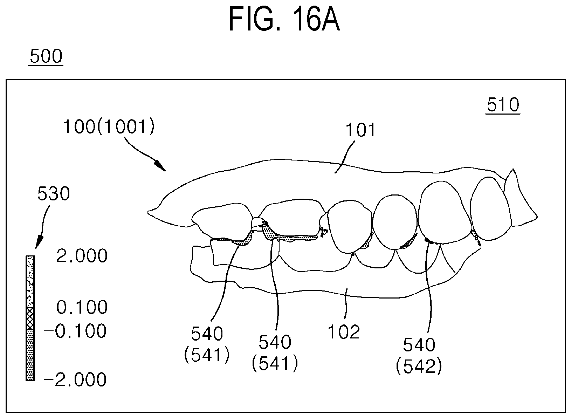

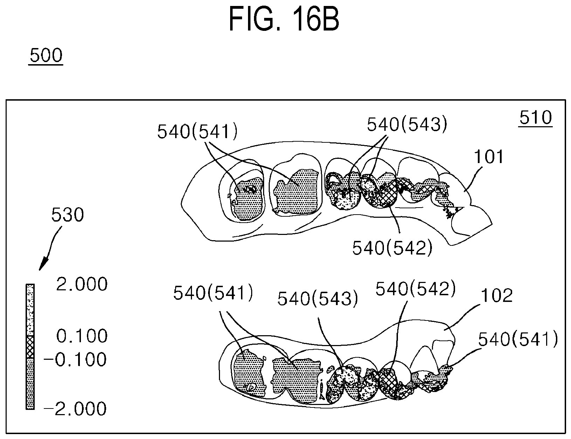

FIG. 16 is a diagram illustrating a process in which occlusion analysis of a first occlusion model 1001 is performed to determine a reference occlusion model in a data processing method according to an embodiment of the present disclosure. More specifically, FIG. 16A shows that the upper jaw data 101 and the lower jaw data 102 occlude with each other as a result of occlusion analysis of the first occlusion model 1001, and FIG. 16B shows that the upper jaw data 101 and the lower jaw data 102 are opened as a result of occlusion analysis of the first occlusion model 1001.

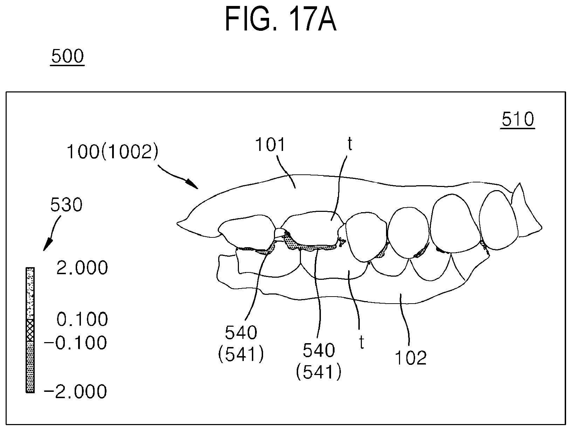

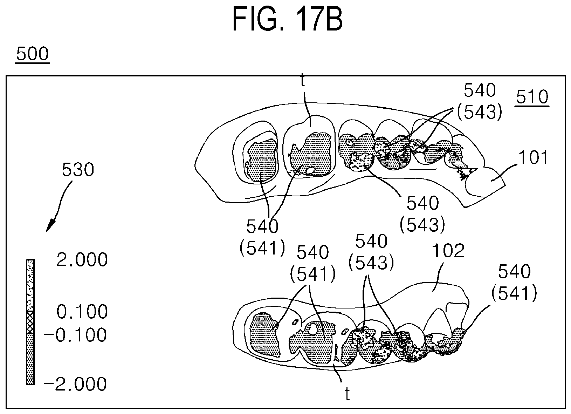

In addition, FIG. 17 is a diagram illustrating a process in which occlusion analysis of a second occlusion model 1002 is performed to determine a reference occlusion model in a data processing method according to an embodiment of the present disclosure. FIG. 17A shows that the upper jaw data 101 and the lower jaw data 102 occlude with each other as a result of occlusion analysis of the second occlusion model 1002, and FIG. 17B shows that the upper jaw data 101 and the lower jaw data 102 are opened as a result of occlusion analysis of the second occlusion model 1002.

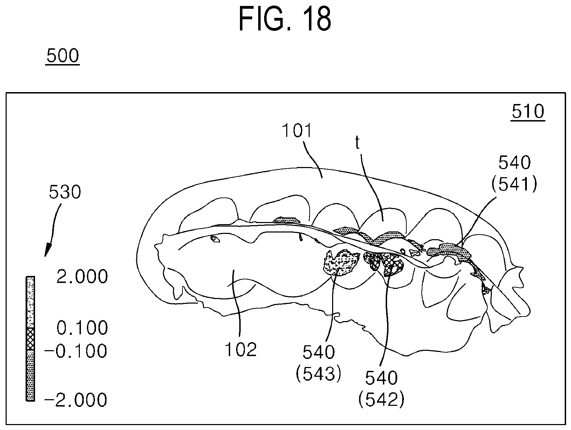

In addition, FIG. 18 is a diagram illustrating a process in which occlusion analysis is performed for an arbitrary occlusion model 100 in a data processing method according to an embodiment of the present disclosure.

Referring to FIGS. 16 and 17, the occlusion analysis unit of the control unit may perform occlusion analysis on the occlusion models 100 generated in the occlusion model generation step S120. The occlusion analysis may be a process of analyzing the occlusion state of each occlusion model 100. Referring to FIGS. 16 and 17, analysis of the first occlusion model 1001 and second occlusion model 1002 may be performed on the model display portion 510 of the screen of the user interface 500. The screen of the user interface 500 may further include an occlusion legend 530, and the occlusion legend 530 may show a numerical range of an occlusion portion 540 representing the degree of occlusion of the upper jaw data 10 1 and the lower jaw data 102. The occlusion portion 540 may include a first occlusion portion 541, a second occlusion portion 542, and a third occlusion portion 543 depending on the degree of occlusion of the upper jaw data 101 and the lower jaw data 102. The first occlusion portion 541 may refer to a portion where the upper jaw data 101 and the lower jaw data 102 have a gap in a first range (e.g., -2.000 to -0.100), the second occlusion portion 541 may refer to a portion where the upper jaw data 101 and the lower jaw data 102 have a gap in a second range (e.g., -0.100 to 0.100), and the third occlusion portion 543 may refer to a portion where the upper jaw data 101 and the lower jaw data 102 have a gap in a third range (e.g., 0.100 to 2.000). The occlusion portions may be divided in more detail or simply as necessary. In addition, the occlusion portions may be displayed to be visually distinguishable using expression elements capable of being easily and visually distinguished, such as predetermined colors and/or predetermined patterns, according to the ranges of the respective occlusion portions.

A reference occlusion model may be determined as an occlusion model 100 having the largest occlusion area among a plurality of occlusion models 100. For example, the reference occlusion model may be determined as an occlusion model 100 having the maximum overall area of the occlusion portion 540. Since the occlusion model 100 having the maximum overall area of the occlusion portion 540 expresses the state in which a number of teeth normally occlude with each other, the occlusion model 100 having the maximum overall area of the occlusion portion 540 may be determined as a reference occlusion model representing a normal bite state.

As another example, the reference occlusion model may be determined as an occlusion model 100 in which the area of the second occlusion portion 542 having a gap in the second range, among the occlusion portions 540, is the maximum. The second occlusion portion 542 may refer to a portion where the tooth data t of the upper jaw data 101 and the tooth data t of the lower jaw data 102 come into close contact with each other. Therefore, as the area of the second occlusion portion 542 increases, it may indicate an occlusion model 100 closer to the normal bite state, and the occlusion model 100 having the maximum area of the second occlusion portion 542 may be determined as a reference occlusion model.

Referring to FIG. 18, the third occlusion portion 543 generated by occlusion of the upper jaw data 101 and the lower jaw data 102 may indicate a portion where the upper jaw data 101 and the lower jaw data 102 overlap each other. An occlusion model 100 in which the upper jaw data 101 and the lower jaw data 102 overlap may be an overbite state. Therefore, even if the overall area of the occlusion portion 540 is the maximum or if the area of the second occlusion portion 542 is the maximum, an occlusion model 100 in which the area of the third occlusion portion 543 is equal to or greater than a predetermined threshold value may be excluded from the reference occlusion model. As described above, by preventing the occlusion model 100 in the overbite state from being determined as the reference occlusion model, the reference occlusion model representing the normal bite state may be accurately determined, and since the remaining occlusion models 100 may be aligned based on a predetermined portion of the reference occlusion model, there is an advantage in which the user is able to easily and visually identify the effective movement according to the switching of the occlusion models 100 through an animation effect.

Although it has been described above that the reference occlusion model determination step S130 is performed based on the occlusion area, the disclosure is not necessarily limited thereto, and an occlusion distance or farthest distance between a front tooth of the tooth data t of the upper jaw data 101 and a front tooth of the tooth data t of the lower jaw data 102 in the occlusion model 100 may be measured so that an occlusion model having the minimum occlusion distance or farthest distance may be determined as the reference occlusion model.

Meanwhile, the occlusion analysis for performing the reference occlusion model determination step S130 may be performed on the tooth data t portion of the upper jaw data 101 and lower jaw data 102, among the occlusion models 100. By performing the occlusion analysis process on the tooth data t portion, unnecessary occlusion analysis on the teeth that do not occlude with each other may be omitted, and the user may quickly acquire the reference occlusion model.

In addition, the upper jaw data 101 described in the present disclosure may include first upper jaw data representing the shape of the upper jaw before processing and second upper jaw data representing the shape of the upper jaw after processing, and the lower jaw data 102 may include first lower jaw data representing the shape of the lower jaw before processing and second lower jaw data representing the shape of the lower jaw after processing, and a plurality of occlusion models 100 may be generated by a combination of any one of the first upper jaw data and the second upper jaw data and any one of the first lower jaw data and the second lower jaw data. In this case, the processing may refer to various procedures performed on the object, and the processing may include at least one of all types of procedures for changing the shapes, positions, and directions of teeth, such as tooth preparation, tooth extraction, reattachment of fractured teeth, and the like.

According to FIGS. 12 to 14, the model selection portion 510 of the screen of the user interface 500 may further include an upper/lower jaw data selection portion 516. The upper/lower jaw data selection portion 516 may include a first upper jaw data selection portion 516a for selecting first upper jaw data, a second upper jaw data selection portion 516b for selecting second upper jaw data, a first lower jaw data selection portion 516c for selecting first lower jaw data, and a second lower jaw data selection portion 516d for selecting second lower jaw data. Through the upper/lower jaw data selection portion 516, the upper jaw data 101 and/or the lower jaw data 102 representing the shapes before or after processing may be aligned with the occlusion data 103 to generate a plurality of occlusion models 100, and the user may easily identify a change in the occlusion state before and after processing.

More specifically, the occlusion model generated by aligning at least one of the second upper jaw data and the second lower jaw data selected by the upper/lower jaw data selection portion 516 with the occlusion data before processing, which is acquired by scanning the buccal side of the object before processing, may express the most ideal fit to which the prosthesis is applied and may be considered when designing the outer surface of the prosthesis (e.g., the contact surface with adjacent teeth and antagonistic teeth). In addition, the occlusion model generated by aligning at least one of the second upper jaw data and the second lower jaw data selected by the upper/lower jaw data selection portion 516 with the occlusion data after processing, which is acquired by scanning the buccal side of the object after processing, may be considered by the user to understand the oral structure of the patient after processing. In addition, the occlusion model generated by aligning the first upper jaw data and the first lower jaw data with the occlusion data before processing, which is acquired by scanning the buccal side of the object before processing, may be considered when designing the inner surface of the prosthesis (e.g., the contact surface with eliminated teeth). In addition, the occlusion model generated by aligning the first upper jaw data and the first lower jaw data and the occlusion data after processing, which is acquired by scanning the buccal side of the object after processing, may represent the degree of overlap or spacing between the upper jaw data (first upper jaw data) and the lower jaw data (first lower jaw data) due to the occlusion power changed after the processing of the object and may be considered when designing the outer surface of the prosthesis.

Therefore, the user may design and manufacture an accurate prosthetic product in consideration of the change in the occlusion state before and after processing, thereby providing optimal treatment to the patient.

The above description is merely an example of the technical idea of the present disclosure, and various modifications and variations may be made by those skilled in the art to which the present disclosure pertains without departing from the essential characteristics of the present disclosure.

Therefore, the embodiments disclosed in the present disclosure are not intended to limit the technical idea of the present disclosure, but to explain the present disclosure, so the scope of the technical idea of the present disclosure is not limited by these embodiments. The protection scope of the present disclosure should be construed according to the claims below, and all technical ideas falling within the equivalent range should be construed as being included in the scope of the present disclosure.

### INDUSTRIAL APPLICABILITY

The present disclosure provides a data processing method of generating a plurality of occlusion models by aligning a plurality of occlusion data with upper jaw data and lower jaw data and continuously displaying at least some of the plurality of occlusion models, thereby improving the user's visual convenience in the process in which the user analyzes the plurality of occlusion models through an animation effect according to switching between the plurality of occlusion models.

## Claims

1. A data processing method comprising:
an occlusion data acquisition step of acquiring a plurality of occlusion data comprising at least a portion of a shape of an upper jaw and a shape of a lower jaw of an object;
an occlusion model generation step of generating a plurality of occlusion models by aligning each of the plurality of occlusion data with upper jaw data representing the upper jaw and lower jaw data representing the lower jaw; and
an occlusion model display step of continuously displaying at least some of the plurality of occlusion models.

2. The data processing method of claim 1, wherein the plurality of occlusion data are acquired by scanning a portion of a buccal side of the object having a plurality of different occlusion states.

3. The data processing method of claim 1, wherein in the occlusion model generation step, after the plurality of occlusion models are generated, corresponding portions of remaining occlusion models are aligned based on a predetermined portion of any one occlusion model among the plurality of occlusion models.

4. The data processing method of claim 1, wherein in the occlusion model generation step, in a state in which one of the upper jaw data and the lower jaw data is fixed, the remaining one is aligned toward the fixed data side.

5. The data processing method of claim 4, wherein the fixed data is the upper jaw data.

6. The data processing method of claim 1, wherein the occlusion model generation step comprises an oral data alignment step comprising
a first alignment step of aligning by moving at least one of the plurality of occlusion data to the upper jaw data side, and
a second alignment step of aligning by moving the lower jaw data to the aligned occlusion data side.

7. The data processing method of claim 1, wherein the occlusion model generation step is repeatedly performed whenever the upper jaw data and the lower jaw data are aligned by applying each of the plurality of occlusion data.

8. The data processing method of claim 1, wherein the occlusion model generation step comprises an occlusion model storage step of storing a shape in which the upper jaw data and the lower jaw data are aligned by any one occlusion data among the plurality of occlusion data.

9. The data processing method of claim 8, wherein the occlusion model generation step further comprises an alignment initialization step of releasing the alignment of the occlusion data with the upper jaw data and the lower jaw data.

10. The data processing method of claim 9, wherein in the alignment initialization step, a position of the upper jaw data and a position of the lower jaw data are returned to positions before the alignment of the occlusion data, or the upper jaw data and the lower jaw data are spaced apart from each other to have a predetermined distance between the upper jaw data and the lower jaw data.

11. The data processing method of claim 10, wherein the lower jaw data is spaced apart from the upper jaw data to have the predetermined distance in a state in which the position of the upper jaw data is fixed.

12. The data processing method of claim 1, wherein the plurality of occlusion models is generated in a single stage on a user interface as the plurality of occlusion data is acquired.

13. The data processing method of claim 1, wherein the occlusion model display step comprises:
a switching model selection step of selecting a second occlusion model different from a first occlusion model from among the plurality of occlusion models;
a movement path acquisition step of acquiring a movement path of the lower jaw data from the first occlusion model to the second occlusion model; and
an animation step of continuously displaying movement of the lower jaw data from the first occlusion model to the second occlusion model along the movement path.

14. The data processing method of claim 13, wherein the occlusion model display step further comprises an interpolation model generation step of generating at least one interpolation model between the first occlusion model and the second occlusion model on the basis of the movement path, and
wherein in the animation step, the movement of the lower jaw data from the first occlusion model to the second occlusion model is continuously displayed by sequentially displaying the first occlusion model, the at least one interpolation model, and the second occlusion model in a predetermined order.

15. The data processing method of claim 1, further comprising a reference occlusion model determination step of determining a reference occlusion model among the plurality of occlusion models, and
wherein the plurality of occlusion models are, based on a predetermined portion of the reference occlusion model, corresponding portions of the remaining occlusion models are aligned.

16. The data processing method of claim 15, wherein the reference occlusion model is determined as an occlusion model having a maximum occlusion area among the plurality of occlusion models.

17. The data processing method of claim 1, wherein the upper jaw data comprises first upper jaw data representing a shape of the upper jaw before processing and second upper jaw data representing a shape of the upper jaw after processing,
wherein the lower jaw data comprises first lower jaw data representing a shape of the lower jaw before processing and second lower jaw data representing a shape of the lower jaw after processing, and
wherein the plurality of occlusion models is generated by any one of the first upper jaw data and the second upper jaw data, and a combination of any one of the first lower jaw data and the second lower jaw data.
